**Europäisches Patentamt**

**European Patent Office**

**Office européen des brevets**

(19)

(11) Veröffentlichungsnummer: **0 180 628**
**B1**

(12) # EUROPÄISCHE PATENTSCHRIFT

(45) Veröffentlichungstag der Patentschrift:
**16.08.89**

(51) Int. Cl.⁴: **A 61 M 5/18**

(21) Anmeldenummer: **85902447.3**

(22) Anmeldetag: **08.05.85**

(86) Internationale Anmeldenummer:
**PCT/DE 85/00149**

(87) Internationale Veröffentlichungsnummer:
**WO 85/05275 (05.12.85 Gazette 85/26)**

(54) INJEKTIONSSPRITZE.

(30) Priorität: **12.05.84 DE 3417756**
**12.05.84 DE 3417757**

(43) Veröffentlichungstag der Anmeldung:
**14.05.86 Patentblatt 86/20**

(45) Bekanntmachung des Hinweises auf die Patenterteilung:
**16.08.89 Patentblatt 89/33**

(84) Benannte Vertragsstaaten:
**GB NL**

(56) Entgegenhaltungen:
**EP-A- 0 037 393**
**WO-A-81/00355**
**DE-A- 1 491 877**
**DE-A- 2 533 594**
**DE-C- 208 720**
**DE-C- 446 818**
**FR-A- 1 051 010**
**FR-A- 1 054 173**
**FR-A- 1 116 922**
**FR-A- 2 191 912**
**US-A- 1 561 854**
**US-A- 4 040 420**
**US-A- 4 109 653**

(73) Patentinhaber: **Lucas, Dieter, Dr., Schlosstrasse 5, D-7763 Öhningen-Kattenhorn (DE)**

(72) Erfinder: **Lucas, Dieter, Dr., Schlosstrasse 5, D-7763 Öhningen-Kattenhorn (DE)**

(74) Vertreter: **Hiebsch, Gerhard F., Dipl.-Ing., Erzbergerstrasse 5A Postfach 464, D-7700 Singen 1 (DE)**

## Beschreibung

Die Erfindung betrifft eine Injektionsspritze mit einer aus einem Nadelraum aus einer Bereitschaftsstellung durch einen Kraftspeicher in eine Betriebsstellung ausschiebbaren Injektionsnadel und mit einem achsparallel neben dem Nadelraum angeordneten, geschlossenen Behälter, mit einer Spritzgutkammer, die eine von einem belastbaren Spritzkolben unter Druck setzbare Injektionsflüssigkeit aufnimmt wobei die einen Nadelkanal aufweisende Injektionsnadel in Betriebsstellung mit der Spritzgutkammer verbunden ist.

Eine solche Injektionsspritze ist aus der DE-A-2 932 719 bekannt. Mit ihr ist es möglich, die Zeitverzögerung zwischen dem Ausschieben der Injektionsnadel und dem Beginn der Injektion länger als bei bekannten Spritzen auszugestalten. Dies wird bei dieser Injektionsspritze dadurch erreicht, dass Nadelraum und Behälter achsparallel verlaufen, dass die Injektionsnadel über eine ihr zugeordnete seitliche Öffnung und einen mit dieser in Betriebsstellung fluchtenden Auslass in einer Wandung des Flüssigkeitsbehälters verbunden ist, wobei der die Flüssigkeit unter Druck setzende Spritzkolben und der Nadelkolben von einer Hebeeinrichtung freigegeben werden, welche die Freigabe des Spritzkolbens gegenüber dem Nadelkolben verzögert.

Aus der DE-A-1 491 877 ist an sich eine Spritzampulle für zwei oder mehrere Spritzflüssigkeiten bekannt. Es sind mehrere Zylinder mit verschiedenen Spritzmedien und zugehörigen Kolben vorhanden. Die Zylinder sind auf der einen Stirnseite mit ihren Kolben mit einem Sammeljoch verbunden. Auf der anderen Stirnseite ist eine Sammelnadel mit der Anzahl der Zylinder entsprechenden Bogennadelanschlüssen vorhanden, die in Stopfen dieser Stirnseite eingestossen werden können. Zuerst werden die Stopfen durchstossen; dann wird über das gemeinsame Sammeljoch auf der anderen Stirnseite die gemischte Injektionsflüssigkeit ausgestossen. Es ist keine Mischkammer vorhanden. An der Verbindungsstelle der Bogennadeln mit der Sammelnadel ist eine gleichmässige Mischung der verschiedenen Spritzmedien nur unter Schwierigkeiten möglich und das Mischungsverhältnis hängt insbesondere davon ab, wie dicht die jeweiligen Kolben in ihren Zylindern sitzen.

Der Erfindung liegt die Aufgabe zugrunde, ausgehend vom eingangs genannten Stand der Technik eine betriebssichere Injektionsspritze zu schaffen, die einen vereinfachten Spritzenaufbau aufweist.

Die Lösung dieser Aufgabe erfolgt dadurch, dass die Spritzgutkammer eine zum Nadelraum hin abragende und diesem gegenüber geschlossene Nebenkammer aufweist, in welche zur Betriebsbereitstellung der Injektionsspritze ein von der Injektionsnadel abragender Schenkel einführbar ist, der mit seinem Nadelkanal in den Nadelkanal der Injektionsnadel mündet.

In der achsparallel neben dem Nadelraum angeordneten Spritzgutkammer steht die Injektionsflüssigkeit betriebsbereit zur Verfügung. Die Spritzgutkammer hat eine Nebenkammer, die zum Nadelraum hin abragt, und in die in Betriebsstellung ein abragender Schenkel der Injektionsnadel einragt. Dadurch kann dieser Schenkel, der mit seinem Nadelkanal in den Nadelkanal der Injektionsnadel mündet, der Injektionsnadel das Spritzgut zuführen. Erst wenn der abragende Schenkel der Injektionsnadel in die Nebenkammer eindringt, ist eine flüssigkeitsdichte Verbindung zum Nadelkanal der Injektionsnadel gegeben. Ein unerwünschtes Aussickern von Spritzgut und Eindringen von Luft in die Injektionsnadel ist unmöglich.

Nach einer besonderen Weiterbildung der Erfindung sind dem Nadelraum innerhalb der Injektionsspritze mehrere Spritzgutkammern zugeordnet, von denen jede mit einem an einer Kolbenstange festliegenden Spritzkolben versehen ist sowie mit einer Nebenkammer in die Bewegungsbahn eines von mehreren Schenkeln der Injektionsnadel einragt.

Beim Eindringen der jeweiligen abragenden Schenkel der Injektionsnadel in ihre zugehörigen Nebenkammern wird das Spritzgut der einzelnen Spritzgutkammern gemischt, wobei die nun miteinander verbundenen Nebenkammern als Mischkammer anzusehen sind. Die Schenkel umgeben die Injektionsnadel kronenartig.

Besondere Weiterbildungen der Erfindung sind in den übrigen Unteransprüchen gekennzeichnet.

Die Erfindung wird in der nachfolgenden Beschreibung bevorzugter Ausführungsbeispiele sowie anhand der Zeichnung näher erläutert; diese zeigt – jeweils Längsschnitt – in

Fig. 1: eine Injektionsspritze mit zwei parallel nebeneinanderliegenden Kammern und einer Injektionsnadel in Bereitschaftsstellung;

Fig. 2: einen Teil der Injektionsspritze nach Fig. 1 mit der Injektionsnadel in Betriebsstellung:

Fig. 3: ein vergrössertes Detail aus Fig. 1 zu einer weiteren Ausführungsform;

Fig. 4: ein anderes Detail aus Fig. 1 in geänderter Ausführung;

Fig. 5: eine Injektionsspritze mit einer Mehrzahl von Kammern um eine zentrale Injektionsnadel.

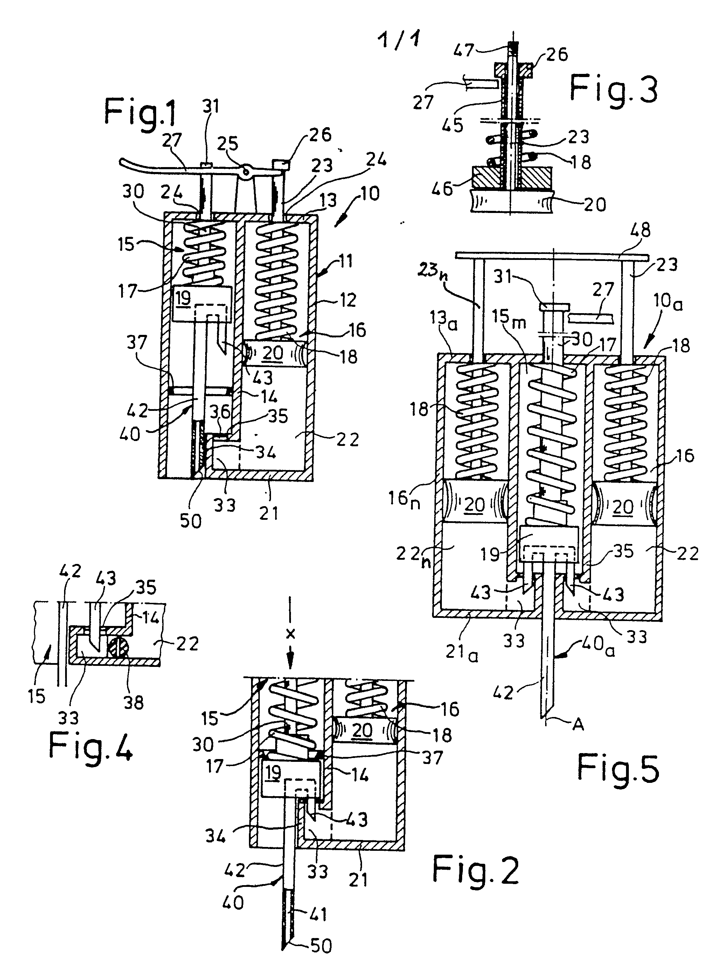

Eine Injektionsspritze 10 weist gemäss Fig. 1 in einem Gehäuse 11 mit Seitenwänden 12 und Deckplatte 13 einen durch eine Mittelwand 14 von einem Nadelraum 15 getrennten achsparallelen Behälter 16 auf. In Nadelraum 15 bzw. im Behälter 16 ist jeweils eine Schraubenfeder 17 bzw. 18 untergebracht, die sich jeweils einerseits gegen jene Deckplatte 13 abstützt sowie anderseits an einen Nadelkolben 19 bzw. einen Spritzkolben 20 anschliesst.

Der Spritzkolben 2 in dem in Fig. 1 rechts angeordneten Behälter 16 begrenzt mit einer Bodenplatte 21 eine Spritzgutkammer 22 für eine aus Gründen der Übersichtlichkeit in der Zeichnung nicht dargestellte Injektionsflüssigkeit. Der Spritzkolben 20 ist an einer Kolbenstange 23 festgelegt, welche eine Ausnehmung 24 der Deckplatte 13 durchsetzt und von einem an der Schwenkstelle 25 schwenkbar gelagerten und einen Stangenkopf 26 untergreifenden Arretierungshebel 27 gehalten wird.

Eine entsprechende Kolbenstange 30 geht vom Nadelkolben 19 des Nadelraumes 15 aus und wird in Arretierungsstellung durch ihren Stangenkopf 31 an jenem Arretierungshebel 27 gehalten.

Am Nadelkolben 19 ist eine U-förmig ausgebildete Injektionsnadel 40 mit durchgehendem Nadelkanal 41 festgelegt bzw. in den Nadelkolben 19 integriert.

Wird die Kolbenstange 30 des Nadelkolbens 19 gelöst, senkt sich der Nadelkolben 19 abwärts und mit ihm geht die daran festliegende Injektionsnadel 40 in ihre Betriebsstellung gemäss Fig. 2. Ein langer Schenkel 42 der Injektionsnadel 40 reicht aus dem unten offenen Nadelraum 15 heraus, während der kurze Schenkel 43 in eine Nebenkammer 33 der rechtsliegenden Spritzgutkammer 22 ragt. Die Nebenkammer 33 schliesst an die Bodenplatte 21 an und steht mit ihrer Wand 34 so weit in den Nadelraum 15 hinein, dass die Wand 34 ein Führungselement für den langen Schenkel 42 der Injektionsnadel 40 anbietet.

Die Decke der Nebenkammer 33 ist mit einem – von einer Haut 35 verschlossenen – Durchbruch 36 versehen. Die Haut 35 kann beim Absenken der Injektionsnadel 40 von deren kurzem Schenkel 42 leicht durchstossen werden. Nach dem Platzieren der Injektionsnadel 40 wird die Kolbenstange 23 des Behälters 16 vom Arretierungshebel 27 gelöst, wodurch die Injektionsflüssigkeit ausgeschoben wird.

Beim Absenken der Injektionsnadel 40 gleitet der Nadelkolben 19 in Ausfahrrichtung x durch einen Lippenring 37 hindurch, der in Betriebsstellung den Nadelkolben 19 als Widerlager oder Anschlag übergreift. Dieser Anschlag 37 kann auch anders ausgeführt sein, muss jedoch das Absenken des Nadelkolbens 19 erlauben und die Funktion des Widerlagers erfüllen.

Dank der besonderen Ausbildung der Injektionsspritze 10 wird bei Anheben bzw. Niederdrücken des Arretierungshebels 27 ein Ansaugen ermöglicht, also ein Unterdruck geschaffen.

Nach Fig. 3 verläuft die Kolbenstange 23 in einem Kolbenrohr 45, das einends mit einer Kolbenscheibe 46 auf dem Spritzkolben 20 aufsitzt und andernends den Stangenkopf 26 trägt; die Kolbenstange 23 ist in dem Kolbenrohr 45 verschiebbar und zum Anschluss an eine nicht dargestellte Handhabe mit einem Endgewinde 47 versehen. Bei dieser Ausführungsform wird die Kraft der Schraubenfeder 18 durch den horizontal geteilten – doppelten – Kolben 20/46 arretiert.

Da die Kolbenstange 23 des Spritzkolbens 20 im Kolbenrohr 45 bewegbar ist, kann ein Spritzvorgang auch nur durch manuellen Druck ermöglicht werden. Hierzu dient die dann angesetzte Handhabe.

Beim Ausführungsbeispiel nach Fig. 4 ist die Nebenkammer 33 durch ein Ventil 38 von der Spritzgutkammer getrennt; durch bewusste Betätigung dieses Ventils 38 wird der Weg der Flüssigkeit zur Injektionsnadel 40 hin freigegeben. Das Ventil 3 ermöglicht somit eine Dosierung des Spritzgutes bzw. der Flüssigkeit.

Die Injektionsspritze 10a nach Fig. 5 enthält mehrere Behälter 16 bis $16_n$ mit Spritzgutkammern 22 bis $22_n$, die um einen zentralen Nadelraum $15_m$ angeordnet und mit gemeinsamer Deck- und Bodenplatte $13_a$ und $21_a$ sowie einem gemeinsamen Druckjoch 48 ihrer Kolbenstangen 23 bis $23_n$ versehen sind.

Die in der Spritzenachse A verlaufende Injektionsnadel $40_a$ weist mehrere – der Anzahl der Spritzgutkammern 22 bis $22_n$ entsprechende – kurze Schenkel 43 auf, die den zentralen langen Schenkel 42 kronenähnlich umgeben und deren Nadelkanäle in den zentralen Nadelkanal 41 des langen Schenkels 42 münden.

Mit dem Durchstossen der Häute 35 mittels der abgeschrägten Nadelschneiden 50 öffnen sich mehrere Spritzgutkammern 22, $22_n$, was ein Vermischen von deren Flüssigkeiten während des Spritzvorganges ermöglicht. Das gemeinsame Druckjoch 48 gewährleistet darüberhinaus eine gleichmässige Vermischung bzw. Verdünnung der verschiedenen Kammersubstanzen.

## Patentansprüche

1. Injektionsspritze mit einer aus einem Nadelraum (15) aus einer Bereitschaftsstellung durch einen Kraftspeicher (17) in eine Betriebsstellung ausschiebbaren Injektionsnadel (40, $40_a$) und mit einem achsparallel neben dem Nadelraum (15) angeordneten, geschlossenen Behälter (16) mit einer Spritzgutkammer (22), die eine von einem belastbaren Spritzkolben (20) unter Druck setzbare Injektionsflüssigkeit aufnimmt wobei die einen Nadelkanal (41) aufweisende Injektionsnadel (40, $40_a$) in Betriebsstellung mit der Spritzgutkammer (22) verbunden ist, dadurch gekennzeichnet, dass die Spritzgutkammer (22) eine zum Nadelraum (15) hin abragende und diesem gegenüber geschlossene Nebenkammer (33) aufweist, in welche zur Betriebsbereitstellung der Injektionsspritze ein von der Injektionsnadel (40, 40a) abragender Schenkel (43) einführbar ist, der mit seinem Nadelkanal in den Nadelkanal (41) der Injektionsnadel (40) mündet.

2. Injektionsspritze nach Anspruch 1, dadurch gekennzeichnet, dass dem Nadelraum ($15_m$) innerhalb der Injektionsspritze ($10_a$) mehrere Spritzgutkammern (22, $22_n$) zugeordnet sind, von denen jede mit einem an einer Kolbenstange (23, $23_n$) festliegenden Spritzkolben (20) versehen ist sowie mit einer Nebenkammer (33) in die Bewegungsbahn eines von mehreren Schenkeln (43) der Injektionsnadel ($40_a$) einragt.

3. Injektionsspritze nach Anspruch 1 oder 2, dadurch gekennzeichnet, dass zwischen der Spritzgutkammer (22) und der Nebenkammer (33) ein steuerbares Sperrorgan (38) vorgesehen ist, bevorzugt ein Ventil.

4. Injektionsspritze nach Anspruch 2 oder 3, dadurch gekennzeichnet, dass die Kolbenstangen (23, $23_n$) durch ein Druckjoch (48) oder eine entsprechende Handhabe verbunden sind.

5. Injektionsspritze nach einem der Ansprüche 1 bis 4, dadurch gekennzeichnet, dass die Injektionsnadel (40) U-förmig ausgebildet ist und neben einem die Injektionsspritze bildenden langen Schenkel (42) einen dazu parallelen kurzen Schenkel (43) aufweist, dessen Nadelkanal (41) in jenen des langen Schenkels (42) mündet.

6. Injektionsspritze nach Anspruch 2 und 5, dadurch gekennzeichnet, dass die Injektionsnadel (40a) mit mehreren um ihre Achse (A) parallel dazu angeordneten kurzen Schenkeln (43) versehen ist, die den zentralen langen Schenkel (42) kronenähnlich umgeben und deren Nadelkanäle in den zentralen Nadelkanal (41) des langen Schenkels (42) münden.

7. Injektionsspritze nach Anspruch 5 oder 6, dadurch gekennzeichnet, dass in der Bewegungsbahn des kurzen Schenkels (43) der Injektionsnadel (40, 40a), eine Sollbruchstelle der Nebenkammer (33) ggf. in Form einer dünnen Haut (35) liegt.

8. Injektionsspritze nach einem der Ansprüche 5 bis 7, dadurch gekennzeichnet, dass der kurze Schenkel (42) der Injektionsnadel (40, 40a) als Nadelscheide (50) endet, und/oder dass der lange Schenkel (42) der Injektionsnadel (40, 40a) in die Achse (A) des Nadelraumes (15, $15_m$) fällt und nahe der die Nebenkammer (33) begrenzenden Wand (34) verläuft, die als Führungsfläche für die Injektionsnadel (40, 40a) dient und dass der kurze Schenkel (43) nahe an den Behälter (16, $16_n$) der Spritzgutkammer (22, $22_n$) anschliessenden Mittelwand (14) verläuft.

9. Injektionsspritze nach einem der Ansprüche 5 bis 8, dadurch gekennzeichnet, dass beide Schenkel (42, 43) der Injektionsnadel (40, 40a) vom an den Kraftspeicher anschliessenden Nadelkolben (19) als Nadelträger abragen.

10. Injektionsspritze nach einem der Ansprüche 1 bis 9, dadurch gekennzeichnet, dass im Nadelraum (15) wenigstens ein Arretierorgan (37) vorgesehen ist, welches den Nadelkolben (19) in Betriebsstellung der Injektionsnadel (40) hält, wobei gegebenenfalls das Arretierorgan (37) als vom Nadelkolben (19) durchfahrbarer flexibler Lippenring (37) ausgebildet ist, der gegen die Ausfahrrichtung (x) der Injektionsnadel (40) als Anschlag dient.

**Revendications**

1. Seringue à injections avec une aiguille à injections (40, 40a) qu'on peut sortir moyennant un accumulateur d'énérgie (17) d'une chambre d'aiguille (15) où elle se trouve dans une position en attente à une position de marche et avec un récipient fermé (16) placé parallèlement à l'axe à côté de la chambre d'aiguille (15) possédant une chambre pour matériau d'injection (22) accueillant un liquide à injections qui peut être mis sous pression par un piston d'injection qu'on peut chargé, l'aiguille à injections (40, 40a) possédant un canal (41) étant liée, dans la position de marche à la chambre pour le matériau d'injection (22) caractérisée en ce que la chambre pour le matériau d'injection (22) présente une chambre secondaire (33) en saillie vers la chambre d'aiguille (15) et fermée envers celle-ci dans laquelle peut être introduite vers la position de marche de la seringue à injections une branche (43) faisant saillie de l'aiguille à injections (40, 40a) qui débouche avec son canal d'aiguille dans le canal d'aiguille (41) de l'aiguille à injections (40).

2. Seringue à injections selon la revendications 1 caractérisée en ce que à la chambre d'aiguille (15m) sont attribués plusieurs chambres pour le matériau d'injection (22, 22n) à l'intérieur de la seringue à injections (10a) dont chacune est pourvue d'un piston d'injection (20) fixé à une barre de piston (23, 23n) et entre, avec une chambre secondaire (33), dans la trajectoire d'une de plusieurs branches (43) de l'aiguille à injections (40a).

3. Seringue à injections selon la revendication 1 ou 2 caractérisée en ce que entre la chambre pour le matériau d'injection (22) et la chambre secondaire (33) est prévu un organe d'arrêt dirigible (38), de préférance une soupape.

4. Seringue à injections selon la revendication 2 ou 3 caractérisée en ce que les barres de piston (23, 23n) sont liées par un joug de pression (48) ou une chose semblable.

5. Seringue à injections selon une des revendications 1 à 4 caractérisée en ce que l'aiguille à injections (40) est formée en U et possède outre une branche longue (42) formant la seringue à injections une branche courte (43) parallèle à celle-ci dont le canal d'aiguille (41) débouche dans celui de la branche longue (42).

6. Seringue à injections selon la revendications 2 et 5 caractérisée en ce que l'aiguille à injections (40a) possède plusieurs branches courtes (43) autour de son axe (A) placées parallèlement à celui-ci qui entourent la branche longue centrale (42) comme un couronnement et dont les canaux d'aiguille débouchent dans le canal d'aiguille centrale (41) de la branche longue (42).

7. Seringue à injections selon la revendication 5 ou 6 caractérisée en ce que dans la trajectoire de la branche courte (43) de l'aiguille à injections (40, 40a) il se trouve un point destiné à la rupture de la chambre secondaire (33), le cas échéant sous forme d'une peau mince (35).

8. Seringue à injections selon au moins une des revendications 5 à 7 caractérisée en ce que la branche courte (42) de l'aiguille à injections (40, 40a) se termine en gaine d'aiguille (50) et/ou que la branche longue (42) de l'aiguille à injections (40, 40a) se trouve dans l'axe (A) de la chambre d'aiguille (15, 15m) et est placée près de la paroi (34) limitant la chambre secondaire (33) et servant de surface de guidage pour l'aiguille à injections (40, 40a) et que la branche courte (43) se trouve près de la paroi au milieu (14) se raccordant au récipient (16, 16n) de la chambre pour matériau d'injection (22, 22n).

9. Seringue à injections selon une des revendications 5 à 8 caractérisée en ce que tous le deux branches (42, 43) de l'aiguille à injections (40,

40a) partent du piston d'aiguille (19) se rattachant à l'accumulateur d'énérgie comme porteurs d'aiguille.

10. Seringue à injections selon une des revendications 1 à 9 caractérisée en ce que dans la chambre d'aiguille (15) est prévu au moins un organe d'arrêt (37) qui tient le piston d'aiguille (19) dans la position de marche de l'aiguille à injections (40), l'organe d'arrêt (37) étant formé, le cas échéant, comme lèvre flexible (37) qui peut être traversée du piston d'aiguille (19) et qui sert comme arrêt contre la direction de sortie (x) de l'aiguille à injections (40).

## Claims

1. Injection syringe with an injection needle (40, 40a) and with a, parallel to the axis and next to the needle chamber (15), placed liquid injection chamber (22) with a container (16), the injection needle (40, 40a) being ejectable and in an operating position from a needle chamber (15), which is in a state of preparedness due to an energy accumulator (17), the needle absorbing an injection liquid by means of a chargeable injection piston (20), which has been placed under pressure, whereby the injection needle (40, 40a), which shows a needle slot (41), is joined to the liquid injection chamber (22) in an operating position, characterized in that the liquid chamber (22), situated next to the needle chamber (15), shows a closed neighbour chamber (33) projecting towards the needle chamber (15) wherein penetrates a projecting thigh (43), of the injection needle (40, 40a) when it is in an operating position, which with its needle slot leads into the needle slot (41) of the injection needle (40).

2. Injection syringe according to claim 1, characterized in that the needle chamber (15m) within the injection syringe (10a) has assigned several liquid injection chambers (22, 22n), each of which is equipped with a injection piston (20) attached to a piston rod (23, 23a), aswell as penetrating with a neighbour chamber (33) into the circle of movement of one or more of the thighs (43) of the injection needle (40a).

3. Injection syringe according to one of the claims 1 or 2, characterized in that between the liquid injection chamber (22) and the neighbouring chamber a steerable blocking organ (38), preferably a valve, is provided.

4. Injection syringe according to claim 2 or 3, characterized in that the piston rods (23, 23a) are connected by a pressure yoke (48) or a respective handle.

5. Injection syringe according to one of the claims 1 to 4, characterized in that the injection needle (40) is developed in a U-form and shows next to the long thigh (42) which forms the injection syringe, a thereto parallel short thigh (43), whose needle slot (41) leads into that of the long thigh (42).

6. Injection syringe according to claim 2 and 5, characterized in that the injection needle (40a) is equipped with several, around her axis and parallel to it, short thighs (43), which surround the central long thigh (42) in a crown-like manner and whose needle slots lead into the central needle slot (41) of the long thigh (42).

7. Injection syringe according to claim 5 or 6, characterized in that in the circle of movement of the short thigh (43) of the injection needle (40, 40a) lies a target point of fracture of the neighbour chamber (33), if necessary in the form of a thin skin (35).

8. Injection syringe according to one of the claims 5 to 7, characterized in that the short thigh (42) of the injection needle (40, 40a) ends as needle sheath (50), and/or that the long thigh (42) of the injection needle (40, 40a) is placed in the axle of the needle chamber (15, 15m) and runs along close to the boardering wall (34) of the neighbour chamber (33), which serves as guidance surface for the injection needle (40, 40a) and that the short thigh (43) runs close to the middle-wall, which joins on to the container (16, 16n) of the liquid injection chamber (22, 22n).

9. Injection syringe according to one of the claims 5 to 8, characterized in that both thighs (42, 43) of the injection needle (40, 40a) project as needle carriers from the needle piston (19), which joins on to the energy accumulator.

10. Injection syringe according to one of the claims 1 to 9, characterized in that for the needle chamber (15) at least one locking organ (37) is provided, which keeps the needle piston (19) in a operating position of the injection needle (40), whereby if necessary the locking organ (37) is formed as a flexible lip ring (37), which can be passed through from the needle piston (19) and which serves as stop motion device against the direction of drive (x) of the injection needle (40).

Fig.1

Fig.2

Fig.3

Fig.4

Fig.5